# EUROPEAN PATENT SPECIFICATION

(11) **EP 1 390 333 B1**
(45) Date of publication and mention of the grant of the patent: **13.10.2004**
(21) Application number: 02704111.0
(22) Date of filing: 11.01.2002
(51) Int. Cl.: C07C 43/295, C07C 317/22, D06M 13/165, D06M 13/268

(54) **PERFLUOROALKYLPHENOL STAIN RESISTS**
PERFLUORALKYLPHENOLSCHMUTZABWEISER
AGENTS ANTI-TACHES A BASE DE PERFLUOROALKYLPHENOL

(30) Priority: 11.01.2001 US 260989 P
(43) Date of publication of application: 25.02.2004
(73) Proprietor: E.I. DUPONT DE NEMOURS AND COMPANY, Wilmington, Delaware 19898 (US)
(72) Inventor: QIU, Weiming, Wilmington, DE 19808 (US)
(74) Representative: Cockerton, Bruce Roger
(86) International application number: PCT/US2002/000843
(87) International publication number: WO 2002/055464

(56) References cited:
- EP-A- 0 190 993
- EP-A- 0 240 601
- EP-A- 0 855 382
- US-A- 3 899 563
- US-A- 4 468 527
- DATABASE WPI Section Ch, Week 8609 Derwent Publications Ltd., London, GB; Class E19, AN 1986-059910 XP002251702 & JP 61 012969 A (TEIJIN), 21 January 1986 (1986-01-21)

## Description

### FIELD OF THE INVENTION

Certain fluoroalkyl substituted phenols are novel and useful as stain resists for synthetic polymer fibers, especially polyamide fibers.

### TECHNICAL BACKGROUND

Some synthetic fibers, such as polyesters and polyamides, are useful as fibers for making various items such as carpets and apparel. Particularly in the case of carpets, which are often fitted and difficult to remove for washing, it is desirable to render these fibers less likely to pick up soil from shoe soles, etc., so that maintenance intervals may be increased and/or soil may be more easily removed. Preferably the fibers should be less likely to pick up both oily and watery soil. This is often done by coating the fiber with a material such as a fluorinated organic compound which renders the fiber both hydrophobic and oleophobic. However coatings have two disadvantages, an extra manufacturing step is needed to coat the fiber, and the coating can sometimes be relatively easily removed by abrasion or other factors. Anti-soil treatments which can be (melt) mixed into the synthetic polymer of the fiber may be more durable and added during the melting and spinning operation, but they preferably have certain miscibility characteristics in the polymer to act as resists.

U.S. Patent 3,899,563 describes certain fluorinated compounds which may be mixed with a synthetic polymer to form a stain resistant fiber. The fluorinated compounds described in this patent are different from those disclosed herein.

U.S. Patents 5,198,151 and 6,011,606 describe the use of certain fluoroalkyl substituted liquid crystal compound useful in liquid crystal displays. The compounds described in both these patents are different from those disclosed herein.

### SUMMARY OF THE INVENTION

This invention concerns a compound of the formula 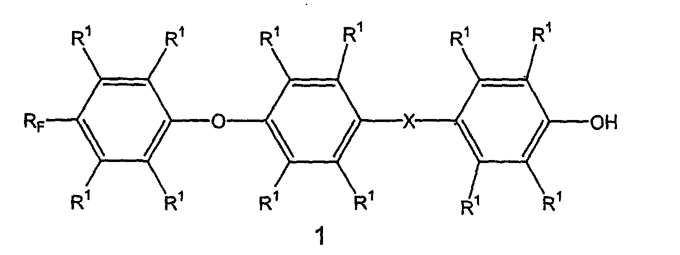 wherein:
each R¹ is independently hydrogen or alkyl containing 1 to 5 carbon atoms;
R_{F} is perfluoroalkyl or perfluoroalkyl containing one or more ether oxygen atoms; and
X is a covalent bond, -SO₂-, -C(CF₃)₂- or -CR²R³-, wherein R² and R³ are each independently hydrogen or alkyl.

Also described herein is a fiber comprising a synthetic polymer and a compound of the formula 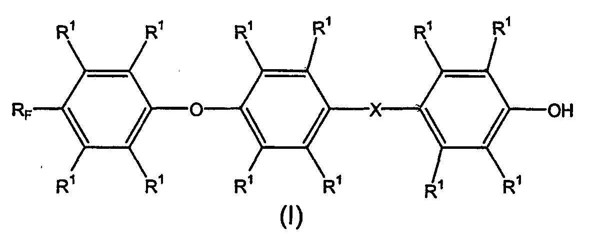 wherein:
each R¹ is independently hydrogen or alkyl containing 1 to 5 carbon atoms;
R_{F} is perfluoroalkyl or perfluoroalkyl containing one or more ether oxygen atoms; and
X is a covalent bond, -SO₂-, -C(CF₃)₂- or -CR²R³-, wherein R² and R³ are each independently hydrogen or alkyl.

### DETAILS OF THE INVENTION

The novel compounds used as anti-soil additives herein have the formula (I). In (I) it is preferred that:
all of R¹ are hydrogen; and/or
R_{F} is perfluoroalkyl containing 16 or fewer carbon atoms, more preferably n-perfluoroalkyl containing 16 or fewer carbon atoms; and/or
R² and R³ are both hydrogen or methyl; and/or
X is a covalent bond or -SO₂-.
   (I) can be made by reacting a p,p'-bisphenol or 4,4'-dihyroxybiphenyl with a p-R_{F} substituted fluorobenzene at higher temperatures in the presence of a base such an alkali metal carbonate, similar to that reported in J.W. Labdie, et al., *Macromolecules*, vol. 23, p. 5371-5373 (1990). The p-perfluoroalkylfluorobenzene can be prepared by the reaction of p-iodofluorobenzene with perfluoroalkylcopper reagent, similar to that reported in G. J. Chen, et al., *J. Fluorine Chem.,* vol. 43, p. 207-228 (1989) (in the paper a bromobenzene was used). These reactions are illustrated in the examples.

   (I) may simply be coated onto a synthetic polymer fiber, but it is preferred that (I) be mixed, for example melt mixed, with the synthetic polymer, and then the synthetic polymer/(I) mixture spun, preferably melt spun, into a fiber. The amount of (I) in the fiber is preferably about 0.01 to about 5 weight percent of the total weight of (I) and the synthetic polymer, more preferably about 0.5 to about 2 weight percent, and/or delivers about 0.5 to about 1 weight percent fluorine. The mixing may be carried out in any suitable apparatus, for example a single or twin screw extruder.

Any synthetic polymer may be used, but preferred synthetic polymers are polyesters, especially poly(ethylene terephthalate) and poly(1,3-propylene terephthalate) which polymers may also include small amount of isophthalic acid residues, aliphatic polyamides, especially nylon-6,6 [poly(hexamethylene adipamide)] and nylon-6 (polycaprolactam), and polyamides are especially preferred.

In the Examples the following abbreviations are used:
DMSO - dimethylsulfoxide
DSC - differential scanning calorimetry
HD - n-hexadecane
mp - melting point
NMP - N-methylpyrrolidone
RT - room temperature
RV - relative viscosity
THF - tetrahydrofuran

### Example 1

### Preparation of 1-Perfluorohexyl-4-fluorobenzene

A mixture of perfluorohexyliodide (67.0 g), 1-bromo-4-fluorobenzene (21.0 g), copper powder (19.0 g), and DMSO (100 ml) was heated at 135°C for 19 h. The solid was filtered and washed with ether (2X20 ml). The ether washes were combined with the filtrates and washed with HCl (aq. 1 N), dried over Na₂SO₄, concentrated, and distilled at reduced pressure to give 1-perfluorohexyl-4-fluorobenzene (28.2 g, bp 77-79°C/270 Pa, 57 % yield). ¹⁹F NMR (CDCl₃) -81.3 (t, J = 10 Hz, 3F), -107.8 (s, 2F), -110.5 (t, J = 15 Hz, 2F), -121.9 (2F), -122.4 (2F), - 123.3 (2F), -126.6 (2F) ppm. ¹H NMR (CDCl₃) 7.21 (dd, J = 9, 5 Hz, 2H), 7.61 (t, J = 9 Hz, 2H) ppm.

### Example 2

### Preparation of 1-Perfluorooctyl-4-fluorobenzene

A mixture of perfluorooctyliodide (164.0 g), 1-bromo-4-fluorobenzene (42.0 g), copper powder (38.0 g), and DMSO (200 ml) was heated at 135°C for 16 h. The solid was filtered and washed with ether (2X20 ml). The ether washes were combined with filtrates and washed with HCl (aq. 1 N), dried over Na₂SO₄, concentrated, and distilled at reduced pressure to give 1-perfluorohexyl-4-fluorobenzene (50 g, bp 84-87°C/270 Pa, 40 % yield). ¹⁹F NMR (CDCl₃) -81.4 (t, J = 10 Hz, 3F), - 107.9 (s, 2F), -110.6 (t, J = 15 Hz, 2F), -121.7 (2F), -122.3 (6F), -123.2 (2F), -126.7 (2F) ppm. ¹H NMR (CDCl₃) 7.21 (dd, J = 9, 5 Hz, 2H), 7.61 (t, J = 9 Hz, 2H) ppm.

### Example 3

### Preparation of 4-(4-Perfluorohexylphenoxy)phenylsulfonylphenol (Ia)

A mixture of 1-perfluorohexyl-4-fluorobenzene (8.28 g), 4,4'-sulfonyldiphenol (10.0 g), potassium carbonate (6.9 g), and NMP (200 ml) was heated at 170°C for 8 h. After being cooled to RT, the reaction mixture was mixed with ether and washed with HCl (1 N) and water, dried over anhydrous Na₂SO₄, and concentrated to give a residue. The residue was purified by flash column chromatography to afford 7.0 g of product, (Ia), yield 54%, mp 134°C. ¹⁹F NMR (CDCl₃) -81.29 (t, J = 9 Hz, 3F), -110.6 (t, J = 15 Hz, 2F), -121.9 (2F), -122.2 (2F), -123.3 (2F), - 126.6 (2F) ppm. ¹³C NMR (CDCl₃, aromatic signals only are listed) 160.4, 160.2, 158.7, 137.2, 132.9, 130.0, 129.8, 129.1 (t, J = 6 Hz), 124.9 (t, J = 25 Hz), 119.5, 119.1, 116.0 ppm. ¹H NMR (CDCl₃) 6.35 (br s, 1H), 6.96 (d, J = 9 Hz, 2H), 7.11 (d, J = 9 Hz, 2H), 7.15 (d, J = 9 Hz, 2H), 7.62 (d, J = 9 Hz, 2H), 7.83 (d, J = 9 Hz, 2H), 7.93 (d, J = 9 Hz, 2H) ppm. Elemental analysis Calc'd C, 44.72; H, 2.02; F, 38.35; Obs'd C, 42.05; H, 1.82; F, 43.35.

### Example 4

### Preparation of 4-(4-Perfluorooctylphenoxy)phenylsulfonylphenol (Ib)

A mixture of 1-perfluorooctyl-4-fluorobenzene (10.0 g), 4,4'-sulfonyldiphenol (9.7 g), potassium carbonate (6.6 g), and NMP (200 ml) was heated at 170°C for 8 h. After being cooled to RT, the reaction mixture was mixed with ether and washed with HCl (1N) and water, dried over anhydrous Na₂SO₄, and concentrated to give a residue, which was purified by flash column chromatography to afford 8.0 g of product, (Ib), yield 57%. DSC mp 151°C. ¹⁹F NMR (CDCl₃) -81.29 (t, J = 9 Hz, 3F), -110.6 (t, J = 15 Hz, 2F), -121.7 (2F), -122.3 (6F), -123.2 (2F), -126.6 (2F) ppm. ¹³C NMR (CDCl₃, only aromatic signals are listed) 160.2 (overlap), 158.7, 137.3, 133.1, 130.0, 129.8, 129.1 (t, J = 6 Hz), 124.9 (t, J = 25 Hz), 119.5, 119.1, 116.2 ppm. ¹H NMR (CDCl₃) 5.95 (br s, 1H), 6.98 (d, J = 9 Hz, 2H), 7.12 (d, J = 9 Hz, 2H), 7.16 (d, J = 9 Hz, 2H), 7.63 (d, J = 9 Hz, 2H), 7.82 (d, J = 9 Hz, 2H), 7.91 (d, J = 9 Hz, 2H) ppm. Elemental analysis: Calc'd C, 41.95; H, 1.76; F, 43.39; Obs'd C, 42.05; H, 1.82; F, 43.35.

### Example 5

### Preparation of 4-(4-Perfluorohexylphenoxy)phenylphenol (Ic)

A mixture of 4,4'-biphenol (9.45 g), NaH (1.5 g), and NMP (300 ml) was heated at 50°C for 3 hours. 1-Perfluorohexyl-4-fluorobenzene (14.0 g) was added to the mixture and the resulting mixture was heated at 170°C for 8 h. After being cooled to room temperature, the reaction mixture was mixed with ether and washed with HCl (1N) and water, dried over Na₂SO₄, concentrated to give a residue. The residue was purified by flash column chromatography to afford 10.9 g of product, (Ic), yield 54%, mp 154°C. ¹⁹F NMR (CDCl₃) -81.29 (t, J = 9 Hz, 3F), -110.3 (t, J = 15 Hz, 2F), -121.9 (2F), -122.3 (2F), -123.3 (2F), -126.6 (2F) ppm. ¹³C NMR (CDCl₃ aromatic signals only are listed) 161.0, 155.1, 154.5, 137.4, 133.1, 128.7 (t, J = 6 Hz), 128.3, 128.2, 120.4, 117.6, 115.8 ppm. ¹H NMR (CDCl₃) 4.75 (br s, 1H), 6.90 (d, J = 9 Hz, 2H), 7.10 (d, J = 9 Hz, 2H), 7.13 (d, J = 9 Hz, 2H), 7.45 (d, J = 9 Hz, 2H), 7.51 (d, J = 9 Hz, 2H), 7.54 (d, J = 9 Hz, 2H) ppm. Elemental analysis: Calc'd C, 49.69; H, 2.25; F, 42.57; Obs'd C, 49.66; H, 2.07; F, 42.62.

### Example 6

### Preparation of 4-(4-Perfluorooctylphenoxy)phenylphenol (Id)

A mixture of 4,4'-biphenol (9.45 g), NaH (1.48 g), and NMP (320 ml) was heated at 50°C for 2 h. 1-Perfluorooctyl-4-fluorobenzene (17.5 g) was added to the mixture and the resulting mixture was heated at 170°C for 8 h. After being cooled to RT, the reaction mixture was mixed with ether and washed with HCl (1N) and water, dried over Na₂SO₄, and concentrated to give a residue. The residue was purified by flash column chromatography to afford 8.0 g of product, (Id), yield 35%, mp 173°C. ¹⁹F NMR (CDCl₃) -81.29 (t, J = 11 Hz, 3F), -110.3 (t, J = 15 Hz, 2F), -121.7 (2F), -122.3 (6F), -123.2 (2F), -126.6 (2F) ppm. ¹³C NMR (CDCl₃ aromatic signals only are listed) 161.0, 155.1, 154.5, 137.4, 133.1, 128.7 (t, J = 6 Hz), 128.3, 128.2, 120.4, 117.6, 115.8 ppm. ¹H NMR (CDCl₃) 4.70 (br s, 1H), 6.90 (d, J = 9 Hz, 2H), 7.10 (d, J = 9 Hz, 2H), 7.13 (d, J = 9 Hz, 2H), 7.45 (d, J = 9 Hz, 2H), 7.51 (d, J = 9 Hz, 2H), 7.54 (d, J = 9 Hz, 2H) ppm. Elemental analysis: Calc'd C, 45.91; H, 1.92; F, 47.48; Obs'd C, 45.91; H, 1.78; F, 47.54

### Example 7

### Preparation of 2-(4-perfluorooctylphenoxyphenyl)-2-(4-hydroxyphenyl)hexafluoroprooane (Ie)

A mixture of 1-perfluorooctyl-4-fluorobenzene (20.0 g), potassium carbonate (13.5 g), 2,2-bis(4-hydroxyphenyl)hexafluoropropane (26.2 g), and NMP (400 ml) was heated at 170°C for 8 h. After being cooled to RT, the reaction mixture was mixed with ether and washed with HCl (1N) and water, dried over Na₂SO₄, and concentrated to give a residue, which was purified by flash column chromatography to afford 20.0 g of product, (Ie), yield 62%, mp 63°C. ¹⁹F NMR (CDCl₃) -81.2 (t, J = 9 Hz, 3F), -110.5 (t, J = 15 Hz, 2F), -121.6 (2F), -122.3 (6F), -123.1 (2F), -126.5 (2F) ppm. ¹³C NMR (CDCl₃ aromatic signals only are listed) 159.7, 156.4, 156.0, 132.1, 131.7, 129.4, 128.9 (t, J = 6 Hz), 125.4, 124.3 (q, J = 289 Hz), 124.0 (t, J = 25 Hz), 118.8, 118.7, 115.2, 63.8 (hep, J =23 Hz) ppm. ¹H NMR (CDCl₃) 4.70 (br s, 1H), 6.80 (d, J = 9 Hz, 2H), 7.02 (d, J = 9 Hz, 2H), 7.09 (d, J = 9 Hz, 2H), 7.30 (d, J = 9 Hz, 2H), 7.42 (d, J = 9 Hz, 2H), 7.54 (d, J = 9 Hz, 2H) ppm. Elemental analysis: Calc'd C, 41.95; H, 1.58; Obs'd C, 42.30; H, 1.38.

### Example 8

In these Examples the following procedures were used:
Solution coating of fibers: A 1% w/v solution of the fluorochemical was dissolved in THF (or other suitable solvent if insoluble in THF), and a monofilament prepared as described below was 'dipped' into this solution. The monofilament was air-dried overnight.
Contact angles: Contact angles of the above monofilaments with water and hexadecane were measured using the well known Wilhelmy method. This is described e.g. in ISBN 0-8247-9046-4 Wettability edited by John C. Berg, published in 1993 by Marcel Dekker, Inc New York, New York 10016. The method is described on pages 13-14 and the geometry used is in Figure 3(c) on page 254. The basic measurement of force used in this method was made using a Dynamic Contact Angle Analyzer, DCA322 system, manufactured by Cahn Instruments Inc. 16207 South Carmenita Road, Cerritos, CA 90701, USA.

Melt Blending: Nylon-66 AF polymer (47 RV, no additives) was dried overnight (16 +/- 1 h) in a vacuum oven at 65°C. Approximately 20 g of polymer chip and the fluorochemical additive were accurately weighed (+/- 0.0001 g) into a glass polymer tube to give an additive concentration of 1% w/w). Five ml of water was then added to the tube. (This is so that steam generation upon heating will displace any oxygen in the tube). The apparatus was constructed as shown with nitrogen flowing directly over the polymer at 600+/-100 ml/min. The vapor bath was heated to temperature and the molten polymer/additive agitated for a further 10+/-1 minutes. The portion of the apparatus highlighted in the diagram was then crash-cooled in liquid nitrogen, breaking the glass tube and leaving the melt blended "slug" exposed for easy removal. The "slug" was allowed to cool under water for 5+/-1 minutes before further processing.

Making Monofilament The polymer "slug" as prepared above was cooled in liquid nitrogen before being broken up in a pulverizer. The polymer pieces thus obtained were again cooled in liquid nitrogen and ground in a 1 mm chip grinder. The powder thus obtained was dried overnight (16 +/- 1 h) in a vacuum oven at 65°C. The extrusion was carried out using a bench top capillary rheometer at a temperature setting of 284°C through a 12.8 mm long x 1.0 mm diameter tungsten carbide die with a polytetrafluoroethylene sealing ring. The sample was introduced to this set-up and extruded under nitrogen pressure onto clean aluminum foil. Monofilament samples of approximately 5 cm length were cut and stored in clean screw top vials.

Contact angle results with various additives are shown in Table 1.

**Table 1**

| **Example** | **Additive** | **Advancing Contact Angles** | | | |
|---|---|---|---|---|---|
| | | **Dip Coated** | | **Melt Mixed** | |
| | | **Water** | **HD** | **Water** | **HD** |
| | | | | | |
| Control (nylon) | none | 71 | 0 | 73 | 0 |
| | | | | | |
| 8 | (Ib) | 82 | 68 | 83 | 80 |
| 9 | (Id) | 91 | 56 | 99 | 54 |
| 10 | (Ia) | 71 | 72 | 64 | 66 |
| 11 | (Ic) | 75 | 56 | 94 | 38 |
| 12 | (Ie) | 73 | | 71 | |

The generally higher advancing contact angles for both water and hexadecane (HD) compared to the control (nylon only) show that most of the fibers with the additives are significantly more hydrophobic and/or oleophobic than the nylon only.

## Claims

1. A compound of the formula 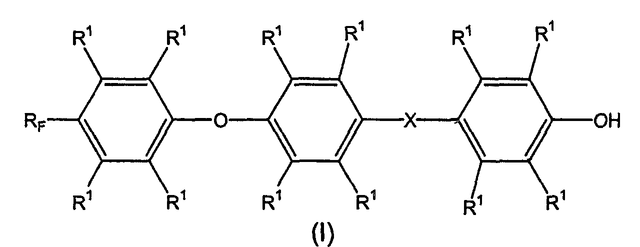 wherein:
each R¹ is independently hydrogen or alkyl containing 1 to 5 carbon atoms;
R_{F} is perfluoroalkyl or perfluoroalkyl containing one or more ether oxygen atoms; and
X is a covalent bond, -SO₂-, -C(CF₃)₂- or -CR²R³-, wherein R² and R³ are each independently hydrogen or alkyl.

2. The compound as recited in Claim 1 wherein all of R¹ are hydrogen.

3. The compound as recited in Claim 2 wherein X is a covalent bond or -SO₂-.

4. The compound as recited in Claim 1 wherein R_{F} is n-perfluoroalkyl containing 16 or fewer carbon atoms.

5. The compound as recited in Claim 3 wherein R_{F} is n-perfluoroalkyl containing 16 or fewer carbon atoms.

6. A fiber comprising a synthetic polymer and a compound of the formula 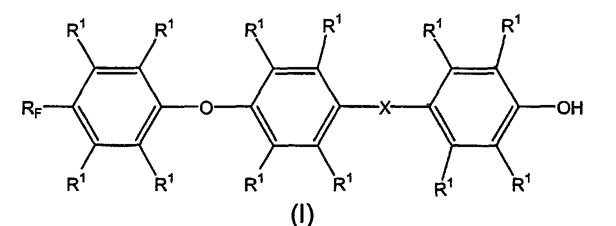 wherein:
each R¹ is independently hydrogen or alkyl containing 1 to 5 carbon atoms;
R_{F} is perfluoroalkyl or perfluoroalkyl containing one or more ether oxygen atoms; and
X is a covalent bond, -SO₂-, -C(CF₃)₂- or -CR²R³-, wherein R² and R³ are each independently hydrogen or alkyl.

7. The fiber as recited in Claim 6 wherein said synthetic polymer is a polyester or a polyamide.

8. The fiber as recited in Claim 7 wherein said synthetic polymer is a polyamide.

9. The fiber as recited in Claim 8 wherein said synthetic polymer is nylon-6,6 or nylon-6.

10. The synthetic fiber as recited in Claim 6 wherein all of R¹ are hydrogen, and R_{F} is n-perfluoroalkyl containing 16 or fewer carbon atoms.

11. The synthetic fiber as recited in Claim 9 wherein all of R¹ are hydrogen, and R_{F} is n-perfluoroalkyl containing 16 or fewer carbon atoms.

12. The synthetic fiber as recited in Claim 10 wherein X is a covalent bond or -SO₂-.

13. The synthetic fiber as recited in Claim 11 wherein X is a covalent bond or -SO₂-.

## Patentansprüche

1. Verbindung der Formel 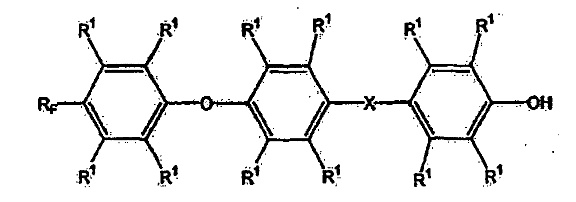 wobei:
jedes R¹ unabhängig Wasserstoff oder Alkyl, enthaltend 1 bis 5 Kohlenstoffatome, ist;
R_{F} Perfluoralkyl oder Perfluoralkyl, enthaltend ein oder mehrere Ethersauerstoffatome, ist; und
X eine kovalente Bindung, -SO₂-, -C(CF₃)₂- oder -CR²R³- ist, wobei R² und R³ jeweils unabhängig Wasserstoff oder Alkyl sind.

2. Verbindung nach Anspruch 1, wobei alle R¹ Wasserstoff sind.

3. Verbindung nach Anspruch 2, wobei X eine kovalente Bindung oder -SO₂- ist.

4. Verbindung nach Anspruch 1, wobei R_{F} n-Perfluoralkyl, enthaltend 16 oder weniger Kohlenstoffatome, ist.

5. Verbindung nach Anspruch 3, wobei R_{F} n-Perfluoralkyl, enthaltend 16 oder weniger Kohlenstoffatome, ist.

6. Faser, umfassend ein synthetisches Polymer und eine Verbindung der Formel 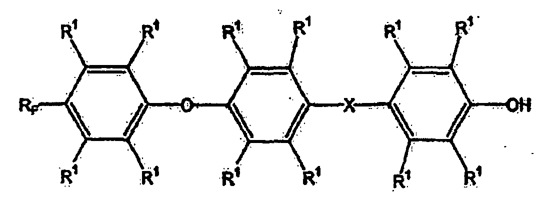 wobei:
jedes R¹ unabhängig Wasserstoff oder Alkyl, enthaltend 1 bis 5 Kohlenstoffatome, ist;
R_{F} Perfluoralkyl oder Perfluoralkyl, enthaltend ein oder mehrere Ethersauerstoffatome, ist; und
X eine kovalente Bindung, -SO₂-, -C(CF₃)₂- oder -CR²R³- ist, wobei R² und R³ jeweils unabhängig Wasserstoff oder Alkyl sind.

7. Faser nach Anspruch 6, wobei das synthetische Polymer ein Polyester oder ein Polyamid ist.

8. Faser nach Anspruch 7, wobei das synthetische Polymer ein Polyamid ist.

9. Faser nach Anspruch 8, wobei das synthetische Polymer Nylon-6,6 oder Nylon-6 ist.

10. Synthetische Faser nach Anspruch 6, wobei alle R¹ Wasserstoff sind, und R_{F} n-Perfluoralkyl, enthaltend 16 oder weniger Kohlenstoffatome, ist.

11. Synthetische Faser nach Anspruch 9, wobei alle R¹ Wasserstoff sind, und R_{F} n-Perfluoralkyl, enthaltend 16 oder weniger Kohlenstoffatome, ist.

12. Synthetische Faser nach Anspruch 10, wobei X eine kovalente Bindung oder -SO₂- ist.

13. Synthetische Faser nach Anspruch 11, wobei X eine kovalente Bindung oder -SO₂- ist.

## Revendications

1. Composé de la formule 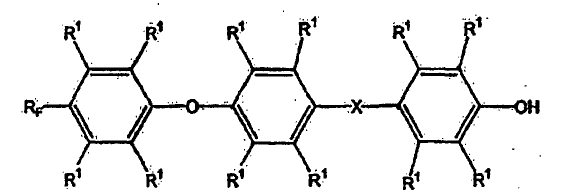 dans laquelle:
chaque R¹ représente indépendamment un hydrogène ou un alkyle contenant 1 à 5 atome(s) de carbone;
R_{F} représente un perfluoroalkyle ou un perfluoroalkyle contenant 1 ou plusieurs atome(s) d'éther oxygène; et
X représente une liaison covalente, -SO₂-, -C(CF₃)₂- ou CR²R³-, dans laquelle R² et R³ représentent chacun indépendamment un hydrogène ou un alkyle.

2. Composé tel qu'énoncé dans la revendication 1, dans lequel tous les R¹ représentent l'hydrogène.

3. Composé tel qu'énoncé dans la revendication 2, dans lequel X représente une liaison covalente ou -SO₂-.

4. Composé tel qu'énoncé dans la revendication 1, dans lequel R_{F} représente un n-perfluroalkyle contenant 16 atomes de carbone ou moins.

5. Composé tel qu'énoncé dans la revendication 3, dans lequel R_{F} représente un n-perfluoroalkyle contenant 16 atomes de carbone ou moins.

6. Fibre comprenant un polymère synthétique et un composé de la formule 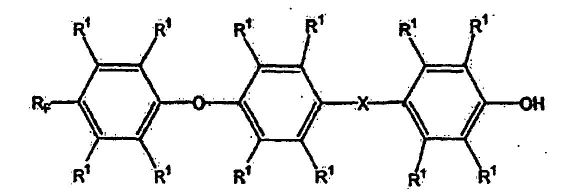 dans laquelle:
chaque R¹ représente indépendamment un hydrogène ou un alkyle contenant 1 à 5 atome(s) de carbone;
R_{F} représente un perfluoroalkyle ou un perfluoroalkyle contenant un ou plusieurs atome(s) d'éther oxygène;
X représente une liaison covalente, -SO₂-, -C(CF₃)₂- ou -CR²R³-, dans laquelle R² et R³ représentent chacun indépendamment un hydrogène ou un alkyle.

7. Fibre telle qu'énoncée dans la revendication 6, dans laquelle ledit polymère synthétique est un polyester ou un polyamide.

8. Fibre telle qu'énoncée dans la revendication 7, dans laquelle ledit polymère synthétique est un polyamide.

9. Fibre telle qu'énoncée dans la revendication 8, dans laquelle ledit polymère synthétique est du nylon-6,6 ou du nylon-6.

10. Fibre synthétique telle qu'énoncée dans la revendication 6, dans laquelle tous les R¹ représentent un hydrogène, et R_{F} représente un n-perfluoroalkyle contenant 16 atomes de carbone ou moins.

11. Fibre synthétique telle qu'énoncée dans la revendication 9, dans laquelle tous les R¹ représentent un hydrogène, et R_{F} représente un n-perfluoroalkyle contenant 16 atomes de carbone ou moins.

12. Fibre synthétique telle qu'énoncée dans la revendication 10, dans laquelle X représente une liaison covalente ou -SO₂-.

13. Fibre synthétique telle qu'énoncée dans la revendication 11, dans laquelle X représente une liaison covalente ou -SO₂-.
